# NEW EUROPEAN PATENT SPECIFICATION

(11) **EP 0 876 805 B2**
(45) Date of publication and mention of the opposition decision: **07.04.2010**
(45) Mention of the grant of the patent: 23.08.2006
(21) Application number: 98303535.3
(22) Date of filing: 06.05.1998
(51) Int. Cl.: A61F 2/06, A61M 25/10

(54) **Intravascular stent and stent delivery system for ostial vessel obstructions**
Intravaskulärer Stent und System zum Einführen (Obstruktion des Ostiums eines Gefässes)
Endoprothèse intravasculaire et système d'introduction pour le traitement de l'obstruction de l'ostium des vaisseaux

(30) Priority: 07.05.1997 US 852547
(43) Date of publication of application: 11.11.1998
(73) Proprietor: Cordis Corporation, Warren, NJ 07040 (US)
(72) Inventor: Turnlund, Todd H., Park City Utah 84098 (US)
(74) Representative: Harris, Ian Richard

(56) References cited:
- EP-A- 0 800 801
- EP-A- 0 830 853
- WO-A-95/32757
- WO-A-96/26689
- WO-A-97/17101
- WO-A-97/25937
- WO-A-97/32543
- WO-A-97/32543
- WO-A-97/40783
- WO-A-98/34668
- US-A- 5 383 892
- US-A- 5 607 444
- US-A- 5 617 878

## Description

This invention is in the field of intravascular stents that are used to maintain patency of a blood vessel.

### BACKGROUND OF THE INVENTION

It has been shown that intravascular stents are an excellent means to maintain the patency of blood vessels following balloon angioplasty. Robert and Tim Fischell in U.S. Pat. No. 4,768,507 describe a helical spring stent design. Palmaz in U.S. Pat. No. 4,733,665 describes a balloon expandable slotted tube stent. Both these designs and others currently known in the art would have fairly uniform stent flexibility before deployment and a reduced radial strength at the proximal and distal ends of the stent after deployment.

Ostial obstructions such as those that often occur at the junction of the aorta and renal artery are difficult to treat with standard angioplasty balloons or stents. Ostial lesions tend to exhibit extreme elastic recoil which makes balloon angioplasty ineffective. Typical stent designs (such as the Palmaz type) have minimal radial strength near each end when deployed. If the stent is allowed to protrude into the aorta so that a more rigid section can engage the ostial lesion, then complications such as stent thrombosis can occur from the bare metal extending out into the blood stream. In addition, the stent mounted on the delivery catheter must often make an almost 90 degree bend to enter the renal artery from the aorta. The longitudinal stiffness of the Palmaz type stent can make this maneuver difficult.

US 5 617 878 describes a method of treating arterial disease at the intersection of two arteries using both a graft and a stent. The stent includes a cylindrical body made from a series of stainless steel "honeycomb" sections. At one end of the body portion is a cylindrical collar having a diameter in excess of that of the body portion.

WO 97 17101 a document which is state of the art according to Art. 54 (3) EPC, describes a specially tapered balloon positioned within a stent. The stent is generally a radial expandable tubular structure, such as a tubular metal mesh. Inflation of the balloon, due to its tapered configuration, inflates a proximal section of the stent into a flared configuration.

EP 0 800 801 a document which is state of the art according to Art. 54 (3) EPC, describes a stent having varied amounts of structural strength along its length. The stent has an open lattice structure and is constructed so that at least one end section has a thicker cross-section and corresponding greater radial strength than the remaining sections of the stent.

WO 97/25937 a document which is state of the art according to Art. 54 (3) EPC, describes a programmable, variably flexible modular stent which includes a plurality of basic circular ring units made from a thin malleable material. Each basic ring unit has a recurring sequence of circumferential segments and circumferential/longitudinal segments. The stent also includes at least one bridging link that longitudinally connects a circumferential segment of each basic ring unit to a corresponding circumferential segment of an adjacent basic ring unit. The basic ring units which are connected to each other by way of the bridging link(s) thereby form a tubular-shaped stent frame.

EP 0,830,853 discloses an intravascular stent for implanting in curved arterial portions or ostial regions. The stent can include an open end region which is fabricated to have a greater radial strength than the remaining axial length of the stent. The stent can also be constructed with an end that has a greater radial strength and sections adjacent the end with greater bending flexibility. The stent has sets of strut members interconnected by "U"-shaped members and the summary section at column 1, lines 45-53, suggests that varied properties can be accomplished by increasing or decreasing the thickness or width of elements of one or more sections relative to other sections and/or increasing or decreasing the actual length of one or more sections and/or changing the cell shape and size and/or changing material properties of the material in one section relative to other sections.

### SUMMARY OF THE INVENTION

The invention is defined in the accompanying claims 1 and 8.

Embodiments of this invention provide a stent and stent delivery system specifically designed for implantation of a stent at the ostium of a vessel. Embodiments of this invention are specifically designed to be able to maintain the patency of lesions such as renal artery ostial obstructions or the ostium of bypass grafts where they are attached to the aorta.

Embodiments of this invention provide a specially designed stent with an increased proximal radial rigidity after expansion and an increased distal flexibility before expansion. In addition this stent may also have the most proximal struts able to be flared out so as not to "hang out" into the aorta. This stent can be combined with a specially designed stent delivery catheter which has a balloon with a tapered shape to allow increased expansion of the proximal section of the stent. This balloon can also produce flaring out of the most proximal stent struts. In addition the combination of radiopaque markers and/or a self-expanding proximal strut attached to the balloon expandable stent could facilitate accurate placement of an ostial stent.

Embodiments of this invention can provide a deployed stent with its proximal section having greater rigidity than the remainder of the stent.

Embodiments of this invention can provide a non-deployed stent with a distal section having more longitudinal flexibility than the remainder of the stent.

Embodiments of this invention include most proximal stent struts capable of being flared out to avoid having metal extend proximal into the vessel's ostium.

Embodiments of this invention include a stent delivery catheter having a balloon which expands to a larger diameter at its proximal end.

Embodiments of this invention include a stent delivery catheter having a balloon for stent delivery which is specially designed to flare out the stent's proximal end struts.

Embodiments of this invention include a stent with radiopaque markers located on the most proximal struts.

These and other objects and advantages of this invention will become obvious to a person of ordinary skill in this art upon reading of the detailed description of this invention including the associated drawings as presented herein.

### BRIEF DESCRIPTION OF THE DRAWINGS

FIG. 1 is a longitudinal cross section of an ostial stent and stent delivery catheter with the delivery balloon expanded.
FIG. 2 is a transverse cross section at 2 - 2 from FIG. 1 for an ostial stent and stent delivery catheter with the delivery balloon expanded.
FIG. 3 is a longitudinal cross section of an ostial stent and stent delivery catheter in place at the ostium of a blood vessel just before deployment of the ostial stent..
FIG. 4 is a longitudinal cross section of an ostial stent and stent delivery catheter in place at the ostium of a blood vessel with the balloon expanded for deployment of the ostial stent.
FIG. 5 is a longitudinal cross section showing the ostial stent in place at the ostium of a blood vessel after the ostial stent has been deployed, the balloon has been deflated and the stent delivery catheter has been removed from the body.
FIG. 6 is a flat lay out view of an ostial stent design in its non-deployed state.
FIG. 7 is a flat lay out view of a structural strut member of the ostial stent shown in FIG. 6.
FIG. 8 is a flat lay out view of an ostial stent design in its deployed state.

### DETAILED DESCRIPTION OF THE DRAWINGS

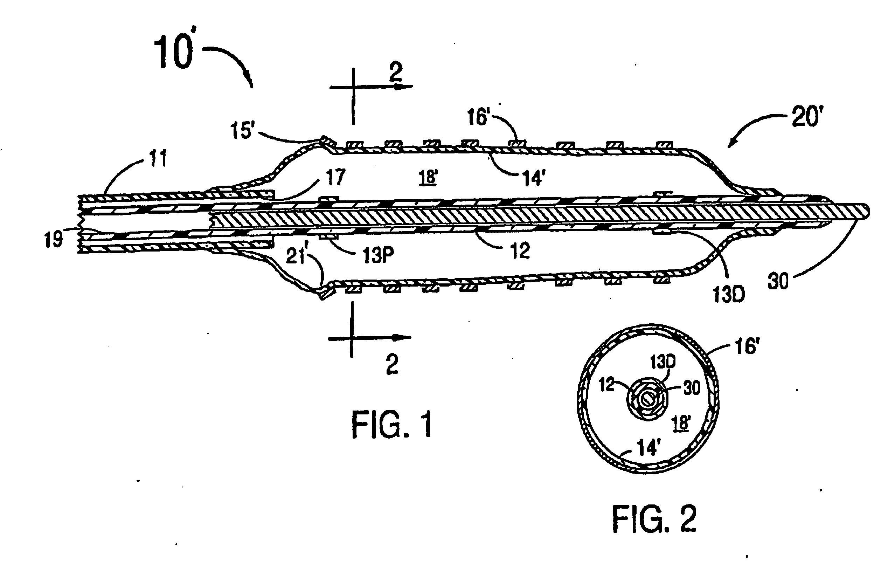

FIG. 1 is a longitudinal cross section of an ostial stent delivery system 10' comprising an ostial stent 16' and a stent delivery catheter 20' having a delivery balloon which is 14' expanded to deploy the stent 16',

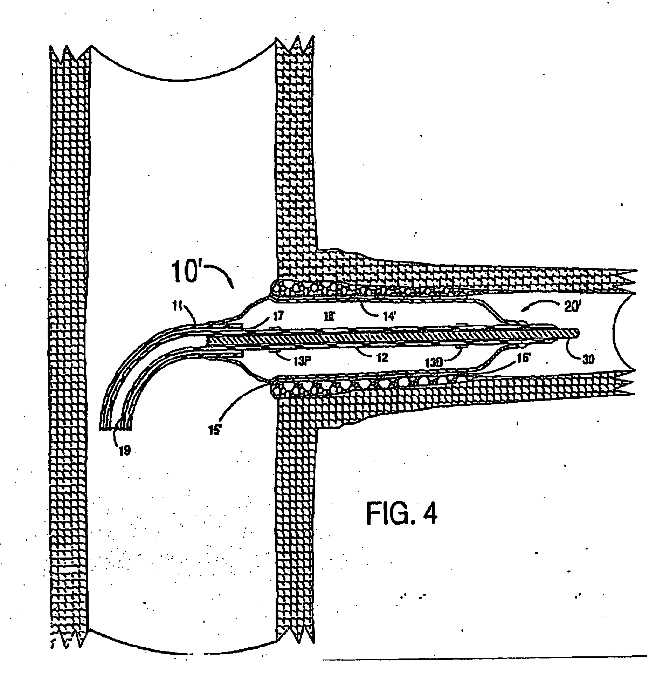

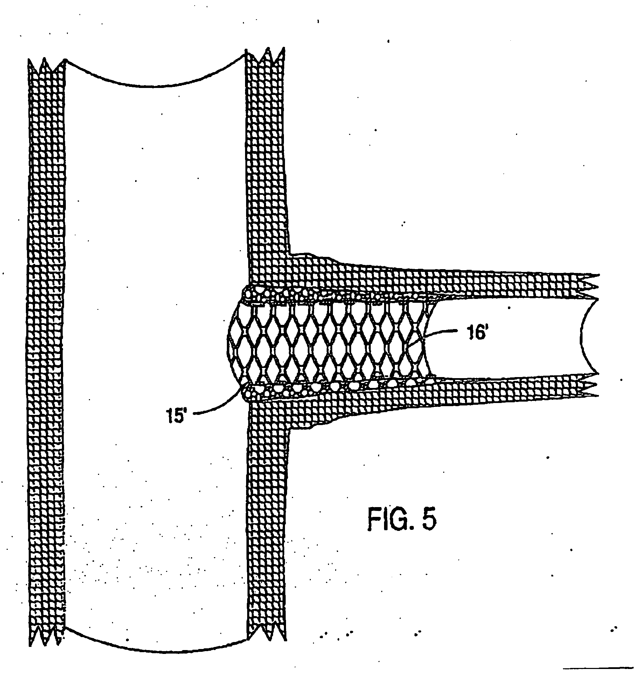

The stent delivery catheter 20' consists of an inner shaft 12, an outer shaft 11, an inflatable balloon 14' that is attached at its proximal end to the outer shaft 11 and at its distal end to the inner shaft 12. An annular passageway 17 that lies between the outer surface of the inner shaft 12 and the inner surface of the outer shaft 11 is in fluid communication with the annular space 18' within the balloon 14', thus allowing inflation and deflation of the balloon 14'. The inner shaft 12 has a central lumen 19 through which to guide wire 30 can be slideably moved. When inflated, the balloon 14' is tapered with a larger diameter at its proximal end compared to its distal end. A bulge 21' near the proximal end of the expanded balloon 14' provides additional expansion to further expand the most proximal strut 15' of the stent 16' to insure that the proximal strut 15' is fully embedded in the arterial wall at the ostium of the implanted vessel as is seen in FIGS. 4 and 5. The bulge 21' may be made of a non-compliant balloon material where there is little change in bulge diameter with balloon inflation pressure or it may be made from a compliant material where it will increase in diameter with inflation pressure to allow for further increases in expansion of the proximal strut 15'. The balloon 14' may be made of a non-compliant plastic which is preformed in the shape shown in FIG. 1 and then folded down over the inner catheter shaft 12. It could also be made of a compliant plastic whose thickness tapers from its thickest at the distal end to its thinnest at the proximal end. For this design, as inflation pressure increases the balloon 14' will expand to a greater diameter at its proximal end than its distal end. What is more, the slope of the taper will increase with higher pressure.

The stent delivery catheter 20' has a proximal radiopaque marker band 13P and a distal radiopaque marker band 13D which generally indicate the proximal and distal extremities of the stent 16' after the balloon 14' is inflated to deploy the stent 16'. It should be noted that in FIG. 1 the wires of the stent 16' are doser together for the proximal section to provide greater radial strength, and are farther apart at the distal section to allow for greater flexibility.

FIG. 2 is a transverse cross section at 2 - 2 from FIG. 1 for the ostial stent delivery system 10' showing the expanded balloon 14' and expanded ostial stent 16'.

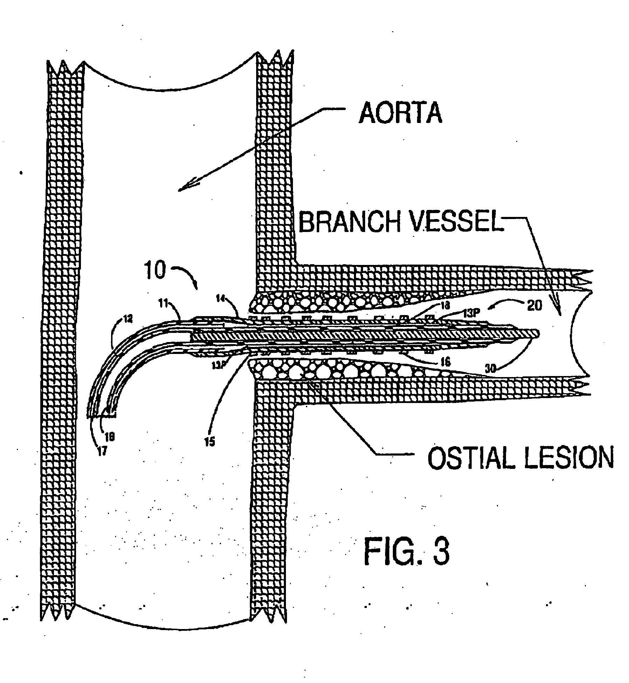

FIG. 3 is a longitudinal cross section of an ostial stent delivery system 10 with stent delivery catheter 20 and non-deployed ostial stent 16 in place over the guide wire 30 at the ostium of a blood vessel just prior to deployment of the ostial stent 16 by inflation of the balloon 14.

FIG. 4 is a longitudinal cross section of the ostial stent delivery system 10' with stent delivery catheter 20' and ostial stent 16', in place over the guide wire 30 at the ostium of a blood vessel after deployment of the ostial stent 16' by inflation of the balloon 14'. Following this step, the balloon 14' is deflated and the stent delivery catheter 20 and guide wire 30 are removed from the body leaving the ostial stent 16', in place at the ostium of the blood vessel as is shown in FIG. 5. It is important to note that the proximal strut 15' of the expanded stent 16' is expanded to a greater diameter than the rest of the stent 16' so as to reduce the probability of the proximal strut 15' extending proximally into the aorta.

FIG. 5 is a longitudinal cross section showing the ostial stent 16' in place at the ostium of a blood vessel after the ostial stent 16' has been deployed, the balloon 14' has been deflated and the stent delivery catheter 20 and guide wire 30 have been removed from the body.

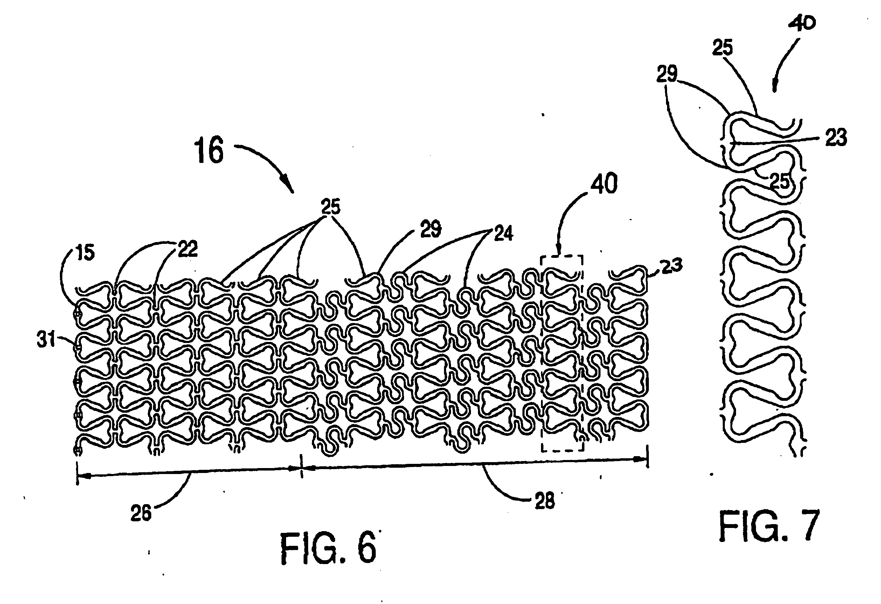

FIG. 6 is a flat lay out view of an ostial stent 16 in its pre-deployment state. The ostial stent 16 is typically laser cut from a stainless steel tube with sets of strut members 40 comprising diagonal struts members 25 with curved ends 29 joined by the circumferential sections 23, shown in an enlarged view in FIG. 7. The sets of strut members 40 are the part of the stent 16 which unfold during expansion and provide the radially rigid structure which maintains vessel patency.

The stent 16 has many sets of strut members 40 which are connected to each other with sets of either longitudinal "H" cross bars 22 or longitudinal "S" undulations 24. The "H" cross bars 22 are shorter in the longitudinal direction than the "S" undulations 24. When the "H" cross bars 22 are used as interconnections, the connected sets of strut members 40 will be closer and more rigidly connected to each other and the radial strength of that section of expanded stent will be increased at the expense of longitudinal flexibility. When the "S" undulations interconnect the sets of strut members 40, the longitudinal flexibility of that section of stent will be increased. The ostial stent 16 of FIG. 6 has a proximal section 26 and distal section 28. The proximal section 26 is composed adjacent sets of strut members 40 having only "H" interconnections 22 to maximize the expanded radial strength. The distal section 28 is composed of adjacent sets of strut members 40 interconnected with only "S" undulations 24 to maximize the longitudinal flexibility. An alternate embodiment (not shown) might have the most distal set of strut members interconnected with "H" cross bars so as to assist the normally radially weak end of the stent. The most proximal strut 15 with radiopaque markers 31 can be given additional expansion during stent deployment as shown in FIGS. 1, 4 and 5.

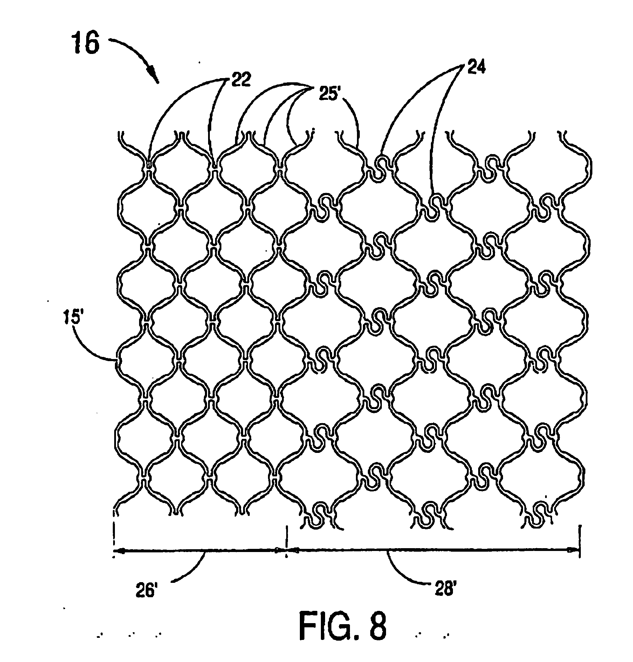

FIG. 8 is a flat lay out view of the ostial stent 16' in its deployed state having deployed diagonal struts 25'. It should be noted here that, in the expanded state, the sets of strut members 40 are closer together and more rigidly connected with the "H" cross bars 22 in the proximal section 26 while the "S" undulations 24 do not as rigidly connect the sets of strut members 40 which are more widely spaced in the distal section 28 of the ostial stent 16'. This design increases the rigidity of the deployed proximal section 26 of the ostial stent 16 while increasing the non-deployed flexibility of the distal section 28.

Although "S" shaped undulations 24 are shown here, it is also envisioned that either one half of an "S" ( a "U") or "S" and "U" undulations connected together or in any combination could also enhance the flexibility of the distal section 28 of the present invention.

Although FIGS. 1 through 4 inclusive illustrate "over-the-wire" types of catheters for delivering a balloon expandable stent, it should be understood that this invention of an ostial stent delivery system could also be used with a "rapid exchange" type of the stent delivery catheter in which the guide wire would exit from the stent delivery catheter near its distal end proximal to the stent location. It is also envisioned that a tubular sheath positioned over the stent and stent delivery catheter could be used in conjunction with this ostial stent 16 and tapered balloon delivery catheter 20. It is also envisioned that an ostial self-expanding stent made of a material such as Nitinol could be produced and heat treated in the shape shown in FIG. 8 then cooled and folded down to the shape in FIG. 6 for introduction into the human body.

The materials of the stent delivery catheter system 10 are well known in the art of devices for interventional cardiology. Typically, all elastomer parts could be made from elastomers such as polyurethane, polyethylene, PTFE, FEP, or any similar plastic. The ostial stent 16 is ideally laser machined from a tube of metal such as stainless steel or tantalum.

Another means to increase the relative radial strength of the proximal end of the stent is to increase the strut width of the bend 29 of the diagonal struts 25. It is also envisioned that having a stent machined from a tube having an increased wall thickness at the proximal end would provide a stent with a greater radial rigidity at its proximal end. Still further it is possible that annealing the distal half of a stent to soften the metal would increase the flexibility of the distal section.

Various other modifications, adaptations, and alternative designs are of course possible in light of the above teachings. Therefore, it should be understood at this time that within the scope of the appended claims the invention may be practiced otherwise than as specifically described herein.

## Claims

1. A stent (16) for maintaining the patency of a vessel of the human body comprising a multiplicity of sets of strut members (40), adjacent sets of strut members being coupled each to the other in a longitudinal extension forming a one piece stent construction, said stent having a proximal section (26) and a distal section (28), said distal section of said stent having a greater longitudinal flexibility prior to deployment when taken with respect to the longitudinal flexibility of said proximal section of said stent and said proximal section having a greater radial rigidity following deployment than said distal section, wherein
adjacent located sets of said strut members in said distal section of said stent are coupled each to the other by at least two substantially longitudinally extending undulating members (24), the substantially longitudinally extending undulating members being formed in one of an S-shaped contour or a U-shaped contour, and
a plurality of substantially linearly directed "H" cross bars connect adjacent sets of strut members in said proximal section of said stent.

2. The stent as recited in claim 1 including at least one radiopaque marker (31) formed on a proximal strut member (15) of the most proximal set of strut members.

3. The stent as recited in claim 1 where said sets of strut members of said distal section have an average dimensional wall thickness smaller than an average dimensional wall thickness of said of sets of strut members of said proximal section stent.

4. The stent as recited in claim 1 where said sets of strut members of said distal section have an average dimensional strut width less than the average dimensional strut width of said sets of strut members of said proximal section of said stent.

5. The stent as recited in claim 1 where said proximal section of said stent has a greater number of sets of strut members per unit length than the distal section.

6. The stent as recited in any one of claims 1 to 5 where said stent is radially expanded responsive to inflation of a balloon onto which balloon the stent is mounted.

7. The stent as recited in any one of claims 1 to 5 where said stent is a radially self-expanding stent.

8. A stent delivery catheter system for placing a stent within a vessel of a human body, the system comprising:
(a) a flexible guide wire (30) extending in a longitudinal direction
(b) a stent delivery catheter (20') having a distal section and a proximal section, said stent delivery catheter having a longitudinally directed central lumen (19) for slideable insertion therethrough of said flexible guidewire, said distal section of said stent delivery catheter including a deployable stent mounted on an expandable balloon (14') having a distal section and a proximal section, said proximal section of said balloon adapted to be expanded to a larger diameter than the diameter of said distal section of said balloon upon inflation of said balloon; and
(c) a stent (16) as recited in any one of claims 1 to 7.

9. The stent delivery catheter system as recited in claim 8 where said proximal section of said balloon is formed of a non-compliant material.

10. The stent delivery catheter system as recited in claim 9 where said proximal section of said balloon is formed of compliant material.

11. The stent delivery catheter system as recited in any one of claims 8 to 10 where the proximal end of said balloon proximal section includes a bulged section (21') having a larger diameter after expansion of said balloon as compared with the remainder of said proximal section of said balloon.

## Patentansprüche

1. Stent (16) zum Aufrechterhalten der Durchgängigkeit eines Gefäßes des menschlichen Körpers mit einer Vielzahl von Gruppen von Strebeteilen (40), wobei benachbarte Gruppen von Strebeteilen jede an der anderen mit einer Längserstreckung unter Bildung eines einstückigen Stentaufbaus gekoppelt sind, wobei der Stent einen proximalen Abschnitt (26) und einen distalen Abschnitt (28) aufweist, wobei der distale Abschnitt des Stents vor der Verwendung eine größere Flexibilität in Längsrichtung aufweist, verglichen mit der Flexibilität in Längsrichtung des proximalen Abschnittes des Stents und wobei der proximale Abschnitt eine größere radiale Festigkeit nach dem Entfalten aufweist als der distale Abschnitt, wobei
benachbarte Gruppen von Strebeteilen in dem distalen Abschnitt des Stents durch mindestens zwei sich im Wesentlichen in Längsrichtung erstreckende wellenförmige Teile (24) miteinander verbunden sind, wobei die sich im Wesentlichen in Längsrichtung erstreckenden wellenförmigen Teile entweder mit einer S-förmigen Kontur oder einer U-förmigen Kontur gebildet sind, und
wobei eine Vielzahl von im Wesentlichen geradlinig gerichteten "H"-Traversen benachbarte Gruppen von Strebeteilen in dem proximalen Abschnitt des Stents verbinden.

2. Stent nach Anspruch 1, mit mindestens einem röntgendichten Marker (31), der auf einem proximalen Strebeteil (15) der proximalsten Gruppe von Strebeteilen gebildet ist.

3. Stent nach Anspruch 1, wobei die Gruppe von Strebeteilen des distalen Abschnittes eine durchschnittliche Außenwanddicke aufweist, die kleiner ist als eine durchschnittliche Außenwanddicke der Gruppe von Strebeteilen des proximalen Abschnittes des Stents.

4. Stent nach Anspruch 1, wobei die Gruppen von Strebeteilen des distalen Abschnittes eine durchschnittliche Außenstrebebreite haben, die kleiner ist als die durchschnittliche Außenstrebebreite der Gruppen von Strebeteilen des proximalen Abschnittes des Stents.

5. Stent nach Anspruch 1, wobei der proximale Abschnitt des Stents eine größere Anzahl von Gruppen von Strebeteilen pro Einheitslänge hat als der distale Abschnitt.

6. Stent nach einem der Ansprüche 1 bis 5, wobei der Stent in Abhängigkeit vom Aufblasen eines Ballons, auf welchem der Stent angebracht ist, radial aufgeweitet ist.

7. Stent nach einem der Ansprüche 1 bis 5, wobei der Stent sich selbst radial aufweitender Stent ist.

8. Stentabgabe-Kathetersystem zum Anbringen eines Stents in einem Gefäß eines menschlichen Körpers, wobei das System aufweist:
(a) einen sich in einer Längsrichtung erstreckenden flexiblen Leitdraht (30);
(b) einen Stentabgabe-Katheter (20') mit einem distalen Abschnitt und einem proximalen Abschnitt, wobei der Stent-Abgabekatheter ein längs gerichtetes mittiges Lumen (19) für das gleitbare Einführen des flexiblen Leitdrahtes durch dieses hat, der distale Abschnitt des Stentabgabe-Katheters einen entfaltbaren Stent einschließt, der auf einem aufweitbaren Ballon (14') angebracht ist mit einem distalen Abschnitt und einem proximalen Abschnitt, wobei der proximale Abschnitt des Ballons geeignet derart ausgestaltet ist, dass er auf einen größeren Durchmesser aufgeweitet wird als der Durchmesser des distalen Abschnittes des Ballons nach dessen Aufblasen; und
(c) einen Stent (16) nach einem der Ansprüche 1 bis 7.

9. Stentabgabe-Kathetersystem nach Anspruch 8, wobei der proximale Abschnitt des Ballons aus einem unnachgiebigen Material gebildet ist.

10. Stentabgabe-Kathetersystem nach Anspruch 9, wobei der proximale Abschnitt des Ballons aus nachgiebigem Material gebildet ist.

11. Stentabgabe-Kathetersystem nach einem der Ansprüche 8 bis 10, wobei das proximale Ende des proximalen Ballonabschnittes einen ausgebauchten Abschnitt (21') einschließt, der nach Aufweiten des Ballons im Vergleich zu dem Rest des proximalen Abschnittes des Ballons einen größeren Durchmesser hat.

## Revendications

1. Stent (16) pour garder ouvert un vaisseau du corps humain, comprenant une multiplicité de jeux d'éléments d'entretoises (40), les jeux adjacents d'entretoises étant accouplés les uns aux autres selon une extension longitudinale formant une construction de stent d'une seule pièce, ledit stent ayant une section proximale (26) et une section distale (28), ladite section distale dudit stent ayant une plus grande flexibilité longitudinale avant déploiement par comparaison avec la flexibilité longitudinale de ladite section proximale dudit stent et ladite section proximale ayant une plus grande rigidité radiale après déploiement que ladite section distale, où
des jeux situés adjacents desdits éléments d'entretoises dans ladite section distale dudit stent sont accouplés l'un à l'autre par au moins deux éléments à ondulations (24) s'étendant sensiblement longitudinalement, les éléments à ondulations s'étendant sensiblement longitudinalement ayant un contour en forme de S ou un contour en forme de U, et
une pluralité de barres transversales en "H" dirigées sensiblement linéairement relient les jeux adjacents d'éléments d'entretoises dans ladite section proximale dudit stent.

2. Stent selon la revendication 1, comprenant au moins un marqueur radio-opaque (31) formé sur un élément d'entretoise proximal (15) du jeu d'éléments d'entretoises le plus proximal.

3. Stent selon la revendication 1, dans lequel lesdits jeux d'éléments d'entretoises de ladite section distale ont une épaisseur de paroi dimensionnelle moyenne inférieure à une épaisseur de paroi dimensionnelle moyenne desdits jeux d'éléments d'entretoises de ladite section proximale du stent.

4. Stent selon la revendication 1, dans lequel lesdits jeux d'éléments d'entretoises de ladite section distale ont une largeur d'entretoise dimensionnelle moyenne inférieure à la largeur d'entretoise dimensionnelle moyenne desdits jeux d'éléments d'entretoises de ladite section proximale dudit stent.

5. Stent selon la revendication 1, dans lequel ladite section proximale dudit stent a un plus grand nombre de jeux d'éléments d'entretoises par unité de longueur que la section distale.

6. Stent selon l'une quelconque des revendications 1 à 5, dans lequel ledit stent est dilaté radialement en réponse au gonflage d'un ballonnet, sur lequel ballonnet est monté le stent.

7. Stent selon l'une quelconque des revendications 1 à 5, dans lequel ledit stent est un stent à auto-dilatation radiale.

8. Système de cathéter d'introduction de stent pour mettre un stent en place au sein d'un vaisseau d'un corps humain, le système comprenant :
(a) un fil-guide flexible (30) s'étendant dans une direction longitudinale ;
(b) un cathéter d'introduction de stent (20') ayant une section distale et une section proximale, ledit cathéter d'introduction de stent comportant une lumière centrale (19) dirigée longitudinalement pour insertion par glissement en son sein dudit fil-guide flexible, ladite section distale dudit cathéter d'introduction de stent comprenant un stent apte à être déployé, monté sur un ballonnet dilatable (14') ayant une section distale et une section proximale, ladite section proximale dudit ballonnet étant apte à être dilatée jusqu'à un plus grand diamètre que le diamètre de ladite section distale dudit ballonnet lors du gonflage dudit ballonnet ; et
(c) un stent (16) tel que mentionné selon l'une quelconque des revendications 1 à 7.

9. Système de cathéter d'introduction de stent selon la revendication 8, dans lequel ladite section proximale dudit ballonnet est faite d'un matériau non souple.

10. Système de cathéter d'introduction de stent selon la revendication 9, dans lequel ladite section proximale dudit ballonnet est faite d'un matériau souple.

11. Système de cathéter d'introduction de stent selon l'une quelconque des revendications 8 à 10, dans lequel l'extrémité proximale de ladite section proximale de ballonnet comprend une section renflée (21') ayant un plus grand diamètre après dilatation dudit ballonnet par comparaison avec le restant de ladite section proximale dudit ballonnet.
